# EUROPEAN PATENT SPECIFICATION

(11) **EP 3 782 691 B1**
(45) Date of publication and mention of the grant of the patent: **09.09.2026**
(21) Application number: 19787582.6
(22) Date of filing: 09.04.2019
(51) Int. Cl.: A61M 25/01

(54) **MOVABLE CATHETER**
BEWEGLICHER KATHETER
CATHÉTER MOBILE

(30) Priority: 16.04.2018 JP 2018078540
(43) Date of publication of application: 24.02.2021
(73) Proprietor: NATIONAL UNIVERSITY CORPORATION SHIGA UNIVERSITY OF MEDICAL SCIENCE, Otsu-shi, Shiga 520-2192 (JP); Zeon Corporation, Tokyo 100-8246 (JP)
(72) Inventor: YAMADA, Atsushi, Otsu-shi, Shiga 520-2192 (JP); TANI, Tohru, Otsu-shi, Shiga 520-2192 (JP); YONEMICHI, Wataru, Tokyo 100-8246 (JP)
(74) Representative: Maiwald GmbH
(86) International application number: PCT/JP2019/015447
(87) International publication number: WO 2019/203061

(56) References cited:
- EP-A1- 3 100 761
- JP-A- 2014 018 391
- JP-A- 2014 018 391
- JP-A- H0 397 428
- JP-A- H0 397 428
- JP-A- H0 966 109
- JP-A- H05 285 089
- JP-A- H1 033 688
- JP-A- H11 267 095
- JP-U- H0 442 838
- US-A1- 2012 190 927
- US-A1- 2016 367 787

## Description

### TECHNICAL FIELD

The present invention relates to a catheter, which is a medical treatment tool used for performing various types of treatments, examinations, and the like. More particularly, the invention relates to a movable catheter (steerable catheter) having a freely deflectable tip portion or the like.

### BACKGROUND ART

A medical treatment tool (such as a catheter for contrast agent injection, an electrode catheter, an ablation catheter, and a catheter sheath) is inserted into target tissue such as various organs (bile duct, heart, and so on) through a body cavity, a lumen, a blood vessel, or the like. A movable catheter is known as the medical treatment tool. The direction of the tip (distal end) of the catheter inserted into the body can be deflected by the operation of an operation portion provided on the base end (proximal end) side of the catheter disposed outside the body so that, for example, the movable catheter can be inserted with ease or is capable of approaching the target tissue with ease (see, for example, Patent Document 1 and Patent Document 2).

The catheter described in Patent Document 1 is an endoscopic catheter used for X-ray contrast agent injection into a bile duct for intra-bile duct examination or the like. The tip portion of the catheter described in Patent Document 1 can be deflected (curved) by an operation wire being operated from the outside of the body such that the catheter is inserted into the duodenum via an endoscope and then easily reaches the bile duct by the tip portion being inserted from the duodenum side into the duodenal papilla. The catheter described in Patent Document 1 has a lumen into which the operation wire for deflecting the tip portion is inserted in addition to a large-diameter lumen used for contrast agent injection or the like. The operation wire is joined to the pointed tip that is provided in the tip portion of the catheter by means such as plasma welding, and thus it is possible to deflect the tip portion of the catheter by pulling the operation wire outside the body.

The catheter described in Patent Document 2, which has a movable tip, is used so that, for example, an ablation catheter is guided to an area to be treated in the heart and a catheter ablation procedure is performed on the heart. The tip portion of the catheter described in Patent Document 2 can be deflected (curved) by an operation portion being operated from the outside of the body such that the tip of the ablation catheter is easily guided to a desired heart position. A catheter tube constituting the catheter described in Patent Document 2 has a main lumen into which various treatment tools are inserted and a pair of wire lumens at positions facing each other at an angle of 180° in the tube wall thereof. Further, the part of the tip portion of the catheter tube to be deflected is set such that, for example, the rigidity thereof decreases in stages toward the tip, the tips of a pair of wires respectively inserted through the wire lumens are connected by means such as laser welding to the ring (pull ring) that is integrally mounted in the tip portion, and the base ends of the pair of wires are connected to the operation portion. Further, it is possible to pull one of the wires, loosen the other wire, and control the direction of the tube tip by operating the operation portion.

However, in the related art such as Patent Document 1 and Patent Document 2, the tip of the wire for deflection operation is connected and fixed to the member mounted in the tip portion of the catheter (pointed tip or pull ring) by means such as plasma welding and laser welding and there is a problem that the number of components is increased by the wire fixing member (pointed tip or pull ring) and the work of mounting the member on the catheter and the work of connecting and fixing the tip of the wire to the member are complicated. In addition, it is necessary to secure a region for providing the member in the structure of the catheter in order to provide the wire fixing member. As a result, there is another problem that the catheter is structurally limited, examples of which include the necessity of reducing the tip opening area of the main lumen.
Prior art US 2016/367787 A1 discloses a catheter guiding system and a method thereto. The steerable guide catheter thereby includes a tip ring at a distal end and one or more pullwires configured to engage with the tip ring. When the pullwires are put in tension the steerable guide catheter is subjected to a curving or turning force.
EP 3 100 761 A1 relates to a medical device. The medical device includes a sheath portion, a pair of operation wires and a turning operation portion in which the sheath portion is bent in a direction corresponding to the pulled one operation wire through a turning operation. JP 2014 018391 A discloses a catheter. The catheter has an operating line fastened by marker, and reinforcement coil wound by outer layer and provided with outer peripheral part of sub lumen, where operating line and reinforcement coil are currently formed with one continuous wire.
Prior art JP H03 97428 A relates to a bending operation device for a tubular insertion tool such as a catheter. A bending operation member made of a shape memory material is attached to a curved portion of the flexible tubular insertion tool, and the bending operation member is deformed.

Further prior art US2012190927A1 discloses a movable catheter provided with a tube having a plurality of spaced apart from each other wire lumens outside a main lumen. A substantial half of a wire is inserted through one of the wire lumens, an intermediate part of the wire is folded back on a distal end portion of the tube and another substantial half of the wire is inserted through another one of the wire lumens.

### CITATION LIST

### PATENT DOCUMENT

Patent Document 1: JP 2002-272675 A
Patent Document 2: JP 2014-188039 A
Patent Document 3: US 2016/367787 A1
Patent Document 4: EP 3 100 761 A1
Patent Document 5: JP 2014 018391 A
Patent Document 6: JP H03 97428 A
Patent Document 7: US 2012/0190927 A1

### SUMMARY OF THE INVENTION

### PROBLEM TO BE SOLVED BY THE INVENTION

The invention has been made in view of such circumstances, and an object thereof is to provide a movable catheter that enables the number of components to be reduced and enables the number of manufacturing steps to be reduced.

### MEANS FOR SOLVING PROBLEM

In order to achieve the above object, a movable catheter according to a first aspect of the invention is proposed having the features of claim 1.

In the movable catheter according to the first aspect of the invention, the wire is folded back in one distal end portion of the wire lumen through which the substantial half on one end portion side thereof is inserted and another distal end portion of the wire lumen through which the substantial half on the other end side thereof is inserted. Accordingly, by simultaneously pulling both end portions (one end portion and the other end portion) of the wire disposed on the proximal end side of the tube, it is possible to apply a force for deflecting the distal end portion of the tube to the distal end portion of the tube. Accordingly, it is possible to deflect the distal end portion of the tube in accordance with the disposition of the pair of wire lumens through which the wire is inserted. Accordingly, there is no need to provide a wire fixing member such as the pointed tip and the pull ring of the related art, and thus the number of components can be reduced. In addition, there is no need to perform mounting work on a wire fixing member with respect to the catheter tube and perform wire connection work with respect to the member, and thus it is possible to reduce the number of manufacturing steps. In addition, there is no need to secure a region for providing a wire fixing member in the structure of the catheter, and thus it is possible to reduce the structural limitation of the catheter.

In the movable catheter according to the first aspect of the invention, the tube comprise three or more wire lumens and the movable catheter comprises two or more wires. With this configuration, it is possible to deflect the distal end portion of the tube in two or more directions.

In the movable catheter according to the first aspect of the invention, a tube that has a single-lumen tube having the main lumen and at least one multi-lumen tube integrally disposed outside the single-lumen tube and having a plurality of the wire lumens can be used as the tube. Although a tube in which a plurality of wire lumens are formed in the tube wall of a single tube having a main lumen may be used as the tube, it is also possible to use the tube in which the multi-lumen tube is disposed outside the single-lumen tube. Then, the productivity of the movable catheter can be improved.

In order to achieve the above object, a movable catheter according to a second aspect of the invention is proposed having the features of claim 4.

In addition, in order to achieve the above object, a movable catheter according to a third aspect of the invention is proposed having the features of claim 5.

In the movable catheters according to the second aspect and the third aspect of the invention, the wire is folded back in the distal end portion of the wire lumen through which the substantial half on one end side thereof is inserted and the substantial half on the other end side thereof is passed through the outside of the tube or the inside of the main lumen. Accordingly, by simultaneously pulling both end portions (one end portion and the other end portion) of the wire disposed on the proximal end side of the tube, it is possible to apply a force for deflecting the distal end portion of the catheter tube to the distal end portion of the catheter tube. Accordingly, it is possible to deflect the distal end portion of the tube in accordance with the disposition of the wire lumen through which the wire is inserted. Accordingly, there is no need to provide a wire fixing member such as the pointed tip and the pull ring of the related art, and thus the number of components can be reduced. In addition, there is no need to perform mounting work on a wire fixing member with respect to the catheter tube and perform wire connection work with respect to the member, and thus it is possible to reduce the number of manufacturing steps. In addition, there is no need to secure a region for providing a wire fixing member in the structure of the catheter, and thus it is possible to reduce the structural limitation of the catheter.

### BRIEF DESCRIPTION OF DRAWINGS

Fig. 1 is a plan view illustrating the appearance configuration of a movable catheter according to an embodiment of the invention;
Fig. 2A is a cross-sectional view taken along line IIa-IIa in Fig. 1;
Fig. 2B is an enlarged perspective view illustrating a main part of the movable catheter of Fig. 1;
Fig. 2C is a cross-sectional view of the sheath main body of the movable catheter of Fig. 2B taken along a plane passing through the central axes of a pair of wire lumens;
Fig. 3A is a perspective view illustrating a case where the number of wires inserted through the wire lumen of the movable catheter of Fig. 2B is increased;
Fig. 3B is a cross-sectional view of the sheath main body of the movable catheter of Fig. 3A taken along a plane orthogonal to the central axis thereof;
Fig. 3C is a cross-sectional view of the sheath main body of the movable catheter of Fig. 3A taken along a plane passing through the central axes of the pair of wire lumens;
Fig. 4A is a diagram illustrating a modification example of the movable catheter of Fig. 3A;
Fig. 4B is a diagram illustrating another modification example of the movable catheter of Fig. 3A;
Fig. 4C is a diagram illustrating still another modification example of the movable catheter of Fig. 3A;
Fig. 5A is an enlarged perspective view illustrating a main part of a movable catheter according to another embodiment of the invention;
Fig. 5B is a cross-sectional view of the sheath main body of the movable catheter of Fig. 5A taken along a plane orthogonal to the central axis thereof;
Fig. 5C is a cross-sectional view of the sheath main body of the movable catheter of Fig. 5A taken along a plane passing through the central axes of a pair of wire lumens;
Fig. 6A is an enlarged perspective view illustrating a main part of a movable catheter according to still another embodiment of the invention;
Fig. 6B is a cross-sectional view of the sheath main body of the movable catheter of Fig. 6A taken along a plane orthogonal to the central axis thereof;
Fig. 6C is a cross-sectional view of the sheath main body of the movable catheter of Fig. 6A taken along a plane passing through the central axes of a pair of wire lumens;
Fig. 7 is a cross-sectional view illustrating the configuration of the sheath main body of the movable catheter according to still another embodiment of the invention; and
Fig. 8 is a cross-sectional view illustrating the configuration of the sheath main body of the movable catheter according to still another embodiment of the invention.

### MODE(S) FOR CARRYING OUT THE INVENTION

Hereinafter, an embodiment of the invention will be specifically described with reference to the drawings. The catheter sheath (movable sheath) as the movable catheter of the present embodiment is a catheter that is inserted prior to an electrode catheter for detecting an electrocardiogram, an ablation catheter for cauterizing an affected area, and the like and guides the electrode catheter, the ablation catheter, and the like when, for example, catheter ablation is performed. Although the catheter sheath will be described below as an example of the movable catheter to which the invention is applied, the invention is also applicable to an electrode catheter, an ablation catheter, a movable endoscopic catheter used for X-ray contrast agent injection into a bile duct for intra-bile duct examination or the like, and other movable catheters.

It should be noted that the catheter ablation is a treatment method for treating cardiac arrhythmia and is a treatment method by which an ablation catheter having a highfrequency electrode in its tip portion is inserted through a blood vessel into the myocardial tissue that has caused the cardiac arrhythmia, coagulative necrosis is caused by the myocardial tissue or its vicinity being cauterized at approximately 60°C to 70°C, and the arrhythmic circuit is interrupted.

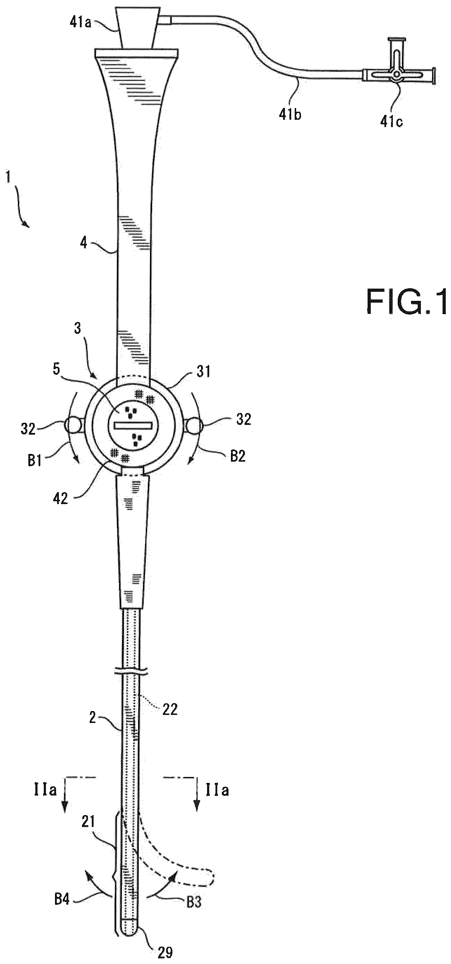

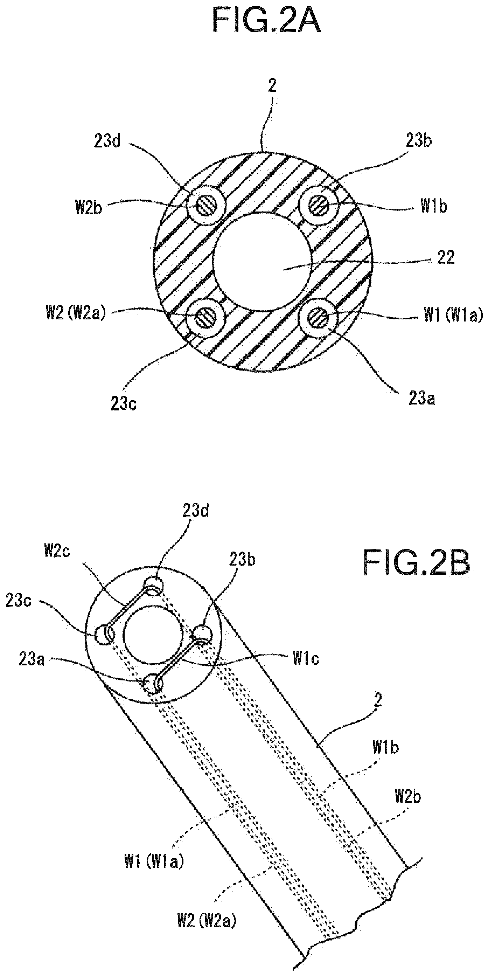

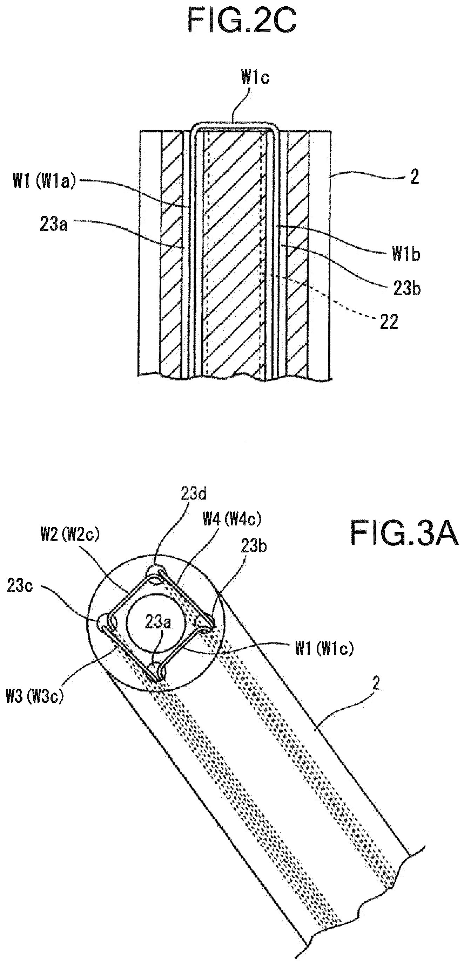

First, refer to Figs. 1 and 2A to 2C. Schematically, a catheter sheath (movable catheter) 1 is configured to include a sheath main body (tube) 2, an operation portion 3, a grip portion 4, and a pair of wires W1 and W2.

The sheath main body 2 is a hollow tube. Although not illustrated, a multilayer tube including a plurality of resin layers and a braid layer made of, for example, reticulated stainless steel is used as the sheath main body 2. A part of the distal end side of the sheath main body 2 is a deflection portion 21 capable of deflecting in any direction. The deflection portion 21 is set to be less rigid (more flexible) than the rest thereof. Further, in the deflection portion 21, the rigidity is set to decrease gradually or in stages toward the tip thereof.

The material of the sheath main body 2 is not particularly limited insofar as the material has flexibility. The material of the sheath main body 2 is preferably a thermoplastic resin or a thermoplastic elastomer. Examples thereof include polyamide-based elastomer such as polyether block amide copolymer, polyamide, polyimide, polyamide-imide, polyethylene terephthalate, polyethylene, polypropylene, polyurethane, ethylene-vinyl acetate copolymer, polyvinyl chloride, polytetrafluoroethylene, tetrafluoroethylene-hexafluoropropylene copolymer, and tetrafluoroethylene-perfluoroalkyl vinyl ether copolymer.

An insertion hole through which the proximal end side part of the sheath main body 2 is inserted is formed in the operation portion 3 and the grip portion 4 attached to the proximal end side of the sheath main body 2. A sheath hub 41a is attached to the proximal end of the grip portion 4.

The sheath hub 41a has a lumen. The sheath main body 2 in the grip portion 4 is attached to the proximal end side of the sheath hub 41a such that the lumen of the sheath hub 41a and a main lumen 22 of the sheath main body 2 communicate with each other. In addition, a catheter insertion port provided with a hemostatic valve is formed on the distal end side of the sheath hub 41a. When the catheter sheath 1 is used (during treatment), the above-mentioned electrode catheter and ablation catheter are inserted from the catheter insertion port of the sheath hub 41a and guided to the main lumen 22 of the sheath main body 2 and the distal end portions thereof are guided to the myocardial tissue to be treated. In addition, a side injection tube is formed in the side portion of the sheath hub 41a and a three-way stopcock 41c is attached to the side injection tube via a tube 41b. It is possible to suction blood in the body or send a liquid medicine into the body by attaching a syringe or the like to the three-way stopcock 41c.

A substantially cylindrical tip protection member 29, which is made of resin and has a hemispherical distal end side, is provided at the distal end of the sheath main body 2 (tip of the deflection portion 21). The tip protection member 29 has a lumen substantially equal in diameter to the main lumen 22 of the sheath main body 2 and is integrally joined (fixed) to the distal end portion of the sheath main body 2 by heat fusion or the like. Alternatively, the tip protection member 29 may be omitted.

In the tube wall of the sheath main body 2, four wire lumens (sub-lumens) 23a to 23d substantially parallel to the main lumen 22 are formed so as to surround the outside of the main lumen 22. The wire lumens 23a to 23d are formed from the proximal end portion of the sheath main body 2 to the distal end portion of the sheath main body 2. The wire lumens 23a to 23d are formed outside the main lumen 22, around the central axis of the sheath main body 2, and apart from each other at an angular pitch (angular interval) of approximately 90°.

In the present embodiment, the wire lumens 23a to 23d are formed so as to open to the distal end surface of the sheath main body 2 to which the tip protection member 29 is joined. Alternatively, the wire lumens 23a to 23d may be formed so as to open to the side surface of the sheath main body 2 near the distal end surface of the sheath main body 2 without reaching the distal end surface of the sheath main body 2 to which the tip protection member 29 is joined. In addition, the distal end of the wire lumen 23a and the distal end of the wire lumen 23b may communicate with each other and the distal end of the wire lumen 23c and the distal end of the wire lumen 23d may communicate with each other near the distal end surface of the sheath main body 2 without the wire lumens 23a to 23d opening to the outside of the sheath main body 2. In this case, these communicating portions may be formed in a substantially U shape so as not to hinder the insertion of the wires W1 and W2.

The single wire W1 is inserted through the wire lumen 23a and the wire lumen 23b, and the single wire W2 is inserted through the wire lumen 23c and the wire lumen 23d. Although the wires W1 and W2 are formed of metal such as stainless steel in the present embodiment, the wires W1 and W2 may be formed of another material such as resin.

A substantial half W1a on one end portion side of the wire W1 is inserted through the wire lumen 23a, an intermediate part W1c thereof is folded back on the distal end surface of the sheath main body 2 to which the tip protection member 29 is joined, a substantial half W1b on the other end portion side thereof is inserted through the wire lumen 23b, and both end portions (one end portion and the other end portion) thereof are disposed so as to be positioned in the operation portion 3 on the proximal end side of the sheath main body 2. Likewise, a substantial half W2a on one end portion side of the wire W2 is inserted through the wire lumen 23c, an intermediate part W2c thereof is folded back on the distal end surface of the sheath main body 2 to which the tip protection member 29 is joined, a substantial half W2b on the other end portion side thereof is inserted through the wire lumen 23d, and both end portions (one end portion and the other end portion) thereof are disposed so as to be positioned in the operation portion 3 on the proximal end side of the sheath main body 2.

Both end portions of the wire W1 and both end portions of the wire W2 are pulled out from the side holes that are provided in the sheath main body 2 in the operation portion 3 provided on the proximal end side of the sheath main body 2 and are connected to the operation portion 3 (a rotation operation member 31). The operation portion 3 has a pair of projection-shaped grip sections 32 and 32 and is held by a holding portion 42 provided on the tip (distal end) side of the grip portion 4 via a screw-type knob member 5.

In the neutral state that is illustrated in Fig. 1, both the wire W1 and the wire W2 are in a tensionless state (or in a state of being tense loosely and substantially evenly) and the deflection portion 21 at the tip of the sheath main body 2 is in a linearly extended state as illustrated in Fig. 1.

When the rotation operation member 31 is rotated in the direction of an arrow B1 in Fig. 1 by the grip sections 32 and 32 of the rotation operation member 31 being appropriately pressed from this neutral state, the wire W1 is pulled and the wire W2 is loosened as the rotation operation member 31 rotates. As a result, the deflection portion 21 at the tip is deflected as indicated by an arrow B3 in Fig. 1.

On the contrary, the wire W1 is loosened and the wire W2 is pulled when the rotation operation member 31 is rotated in the direction of an arrow B2 in Fig. 1 by the grip sections 32 and 32 of the rotation operation member 31 being appropriately pressed. As a result, the deflection portion 21 at the tip is deflected as indicated by an arrow B4 in Fig. 1.

In a case where it is desired to fix the shape of the deflection portion 21 with the deflection portion 21 deflected, the rotation operation member 31 is pressed by the holding portion 42 by the knob member 5 being rotated clockwise and tightened, the rotation operation member 31 is fixed at the current position, and the shape of the deflection portion 21 is fixed. In a case where it is desired to release the fixing of the shape of the deflection portion 21 (in a case where it is desired to adjust the state of deflection), the knob member 5 is rotated counterclockwise and loosened contrary to be above, and then the rotation operation member 31 is loosely pressed by the holding portion 42 and the rotation operation member 31 becomes rotatable. As a result, the fixing of the shape of the deflection portion 21 is released and it is possible to adjust the state of deflection of the deflection portion 21 by gripping the grip section 32 and rotating the rotation operation member 31.

In the embodiment described above, the wire W1 is folded back in the distal end portion of the wire lumen 23a through which the substantial half W1a on one end portion side thereof is inserted and the distal end portion of the wire lumen 23b through which the substantial half W1b on the other end side thereof is inserted. Accordingly, by pulling both end portions of the wire W1 disposed on the proximal end side of the sheath main body 2, it is possible to apply a force for deflecting the distal end portion of the sheath main body 2 to the distal end portion of the sheath main body 2. Accordingly, it is possible to deflect the distal end portion (deflection portion 21) of the sheath main body 2 in accordance with the disposition of the pair of wire lumens 23a and 23b through which the wire W1 is inserted with respect to the central axis of the sheath main body 2. The same applies to the wire W2.

Accordingly, there is no need to provide a wire fixing member such as the pointed tip and the pull ring of the related art, and thus the number of components can be reduced. In addition, there is no need to perform mounting work on a wire fixing member with respect to the catheter tube and perform wire connection work with respect to the member, and thus it is possible to reduce the number of manufacturing steps. In addition, there is no need to secure a region for providing a wire fixing member in the structure of the catheter sheath 1, and thus it is possible to reduce the structural limitation of the catheter sheath 1. For example, it is possible to increase the opening area of the distal end (tip) of the sheath main body 2 (main lumen 22).

In addition, a force for deflecting the deflection portion 21 is applied by both the substantial half W1a of the wire W1 inserted through the wire lumen 23a and the substantial half W1b inserted through the wire lumen 23b being pulled. Accordingly, it is possible to apply the force to a relatively wide range in the circumferential direction of the sheath main body 2 in accordance with the interval (angular interval) between the wire lumen 23a and the wire lumen 23b. The same applies to the wire W2. As a result, the force that is applied to the wire and the risk of wire breakage that is entailed by the deflection operation are smaller than in the related art in which the deflection operation is performed by the single unfolded wire inserted through a single lumen being pulled.

Although the wire W1 and the wire W2 that are used in the embodiment described above have the same specifications such as the same wire diameter (cross-sectional area), the specifications of the wire W1 and the wire W2 may differ from each other. For example, the wire W1 may be larger in wire diameter (cross-sectional area) than the wire W2.

In addition, although the wire lumens 23a to 23d are arranged at an equal angular pitch (90° pitch) in the embodiment described above, the wire lumens 23a to 23d may be arranged at different angular intervals without having to be arranged at the equal angular pitch. By appropriately setting the angular intervals, it is possible to appropriately set the curvature of the deflection portion 21 or the direction of deflection of the deflection portion 21.

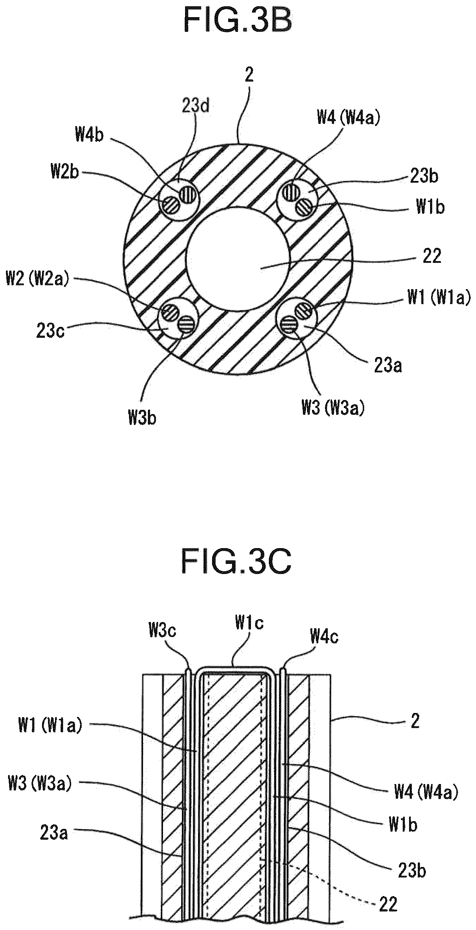

Further, although the two wires of the wire W1 inserted through the wire lumen 23a and the wire lumen 23b and the wire W2 inserted through the wire lumen 23c and the wire lumen 23d are used in the embodiment described above, a configuration in which four wires are used with a wire W3 and a wire W4 added may be adopted as illustrated in Figs. 3A to 3C.

In other words, a substantial half W3a on one end portion side of the wire W3 is inserted through the wire lumen 23a, an intermediate part W3c thereof is folded back on the distal end surface of the sheath main body 2 to which the tip protection member 29 is joined, and a substantial half W3b on the other end portion side thereof is inserted through the wire lumen 23c. A substantial half W4a on one end portion side of the wire W4 is inserted through the wire lumen 23b, an intermediate part W4c thereof is folded back on the distal end surface of the sheath main body 2 to which the tip protection member 29 is joined, and a substantial half W4b on the other end portion side thereof is inserted through the wire lumen 23d. With such a configuration, it is possible to perform deflection in four directions by appropriately selecting and pulling one of the wires W1 to W4. In addition, it is possible to deflect the deflection portion 21 by 360° in any direction by appropriately selecting a combination of two adjacent wires from the wires W1 to W4 and adjusting the balance of the forces that respectively pull the wires. It should be noted that it is necessary to appropriately change the configuration of the operation portion 3 in response to the addition of the wires W3 and W4, examples of which include the addition of a rotation operation member similar to the rotation operation member 31 in the operation portion 3.

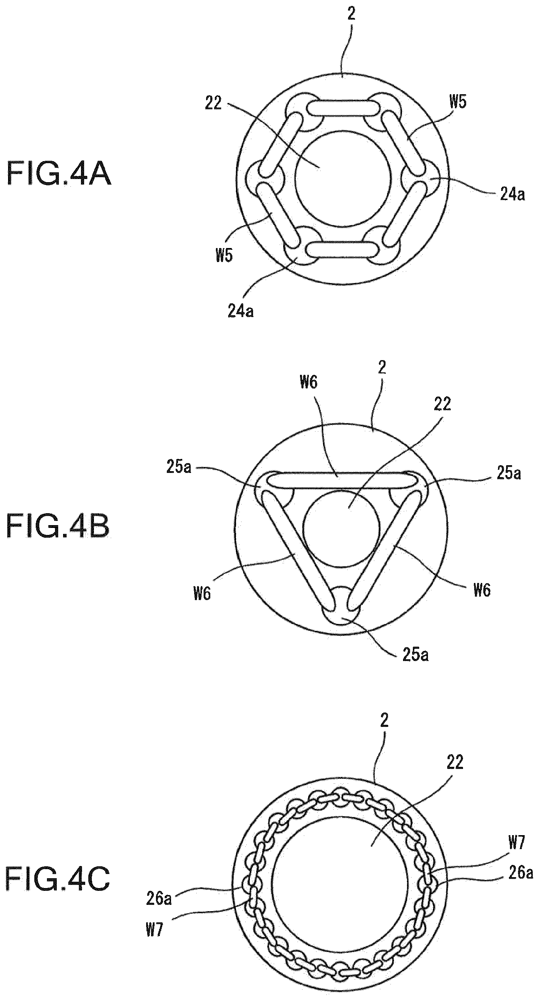

In addition, although a case where the four wire lumens 23a to 23d and the four wires W1 to W4 are provided has been described in the example illustrated in Figs. 3A to 3C, the number of wire lumens can be increased or decreased and the number of wires can be accordingly increased or decreased as illustrated in, for example, Figs. 4A to 4C.

In other words, in Fig. 4A, six wire lumens 24a are provided at an angular pitch of 60° and six wires W5 are provided. As a result, the deflection portion 21 can be deflected in six directions in a case where the wires W5 are pulled one by one and it is possible to deflect the deflection portion 21 by 360° in any direction by appropriately selecting a combination of two adjacent wires W5 and adjusting the balance of the forces that respectively pull the wires. In Fig. 4B, three wire lumens 25a are provided at an angular pitch of 120° and three wires W6 are provided. As a result, the deflection portion 21 can be deflected in three directions in a case where the wires W6 are pulled one by one and it is possible to deflect the deflection portion 21 by 360° in any direction by appropriately selecting a combination of two adjacent wires W6 and adjusting the balance of the forces that respectively pull the wires. In Fig. 4C, 24 wire lumens 26a are provided at an angular pitch of 15° and 24 wires W7 are provided. As a result, the deflection portion 21 can be deflected in 24 directions in a case where the wires W7 are pulled one by one. These are examples, the number of wire lumens may be two or more, and the number of wires may be one or more.

Although the number of wire lumens and the number of wires are equal to each other in each of the examples illustrated in Figs. 4A to 4C, the numbers may be different from each other. For example, the number of wires may be smaller than the number of wire lumens. In addition, although both end portions of the wire are inserted through the pair of wire lumens that are adjacent to each other in each of the examples illustrated in Figs. 4A to 4C, one pair of wire lumens may be, for example, intermittently selected and both end portions of the wire may be inserted therethrough.

It should be noted that two wire lumens and one wire may be provided although this is not illustrated. Then, the deflection portion 21 can be deflected in one direction. In this case, it is preferable that the wire lumens are disposed at an angular interval of less than 180°. Then, the distal end portion of the sheath main body 2 can be easily deflected toward the pair of wire lumens through which the wire is inserted when both end portions of the wire are pulled at the same time.

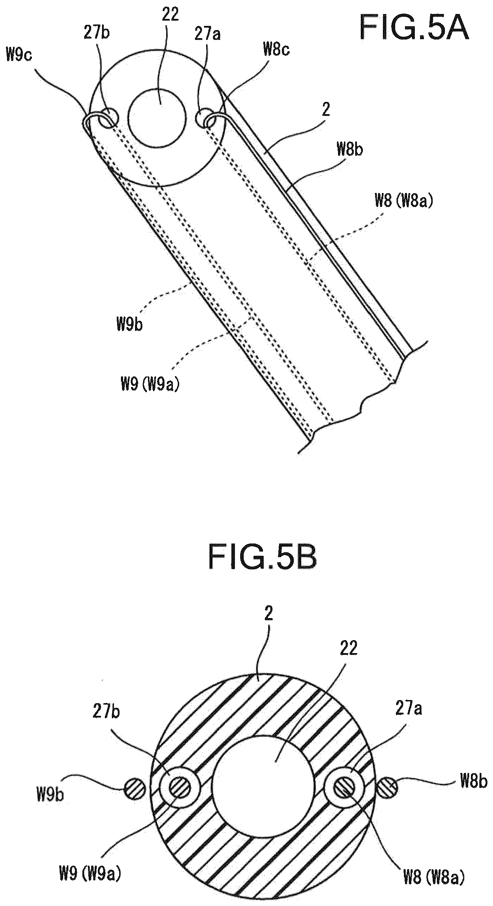

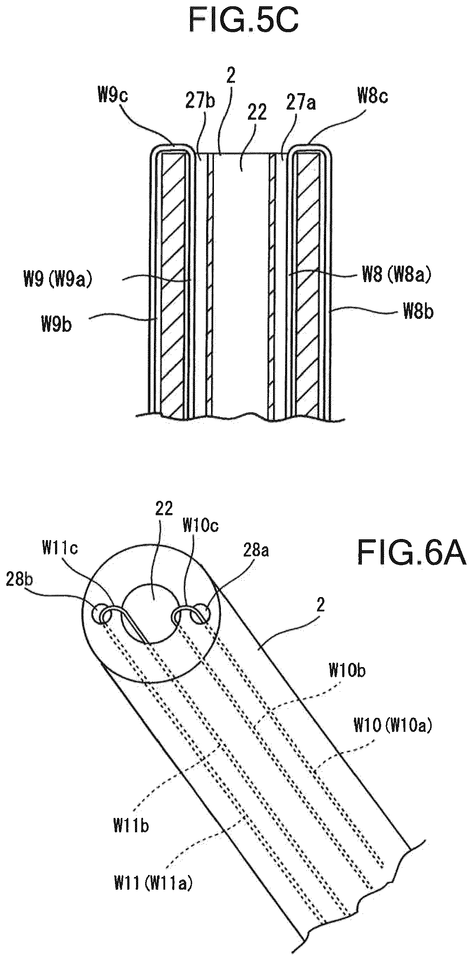

In addition, although the substantial half W1a on one end portion side of the wire W1 and the substantial half W1b on the other end portion side of the wire W1 are inserted through the pair of wire lumens 23a and 23b in the embodiment described above, the configuration that is illustrated in Figs. 5A to 5C may also be adopted. In other words, in Figs. 5A to 5C, a substantial half W8a on one end portion side of a wire W8 is inserted through a wire lumen 27a formed in the sheath main body 2, an intermediate part W8c of the wire W8 is folded back at the distal end of the sheath main body 2, a substantial half W8b on the other end portion side of the wire W8 is passed through the outside of the sheath main body 2, and both end portions (one end portion and the other end portion) thereof are disposed on the proximal end side of the sheath main body 2. Likewise, a substantial half W9a on one end portion side of a wire W9 is inserted through a wire lumen 27b formed in the sheath main body 2, an intermediate part W9c of the wire W9 is folded back at the distal end of the sheath main body 2, a substantial half W9b on the other end portion side of the wire W9 is passed through the outside of the sheath main body 2, and both end portions (one end portion and the other end portion) thereof are disposed on the proximal end side of the sheath main body 2. According to this example illustrated in Figs. 5A to 5C, it is possible to reduce the number of wire lumens while achieving the same action and effect as the configuration illustrated in Figs. 2A to 2C.

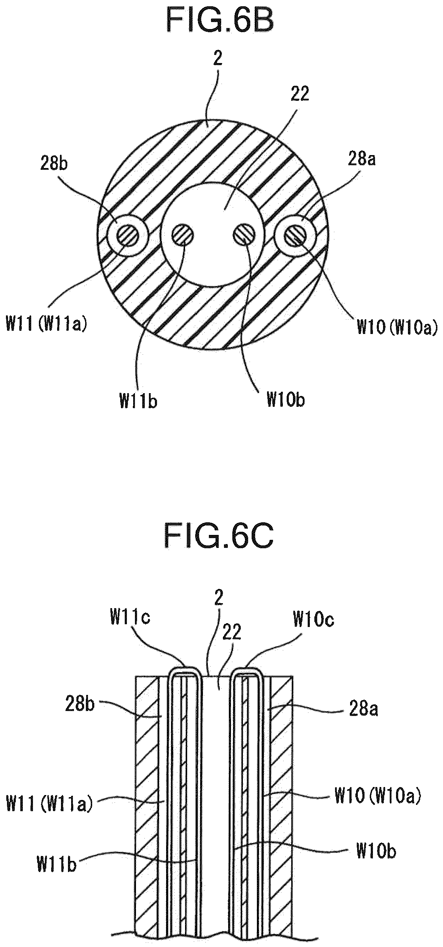

Further, the catheter sheath may be configured as illustrated in Figs. 6A to 6C. In other words, in Figs. 6A to 6C, a substantial half W10a on one end portion side of a wire W10 is inserted through a wire lumen 28a formed in the sheath main body 2, an intermediate part W10c of the wire W10 is folded back at the distal end of the sheath main body 2, a substantial half W10b on the other end portion side of the wire W10 is inserted through the main lumen 22 formed in the sheath main body 2, and both end portions (one end portion and the other end portion) thereof are disposed on the proximal end side of the sheath main body 2. Likewise, a substantial half W11a on one end portion side of a wire W11 is inserted through a wire lumen 28b formed in the sheath main body 2, an intermediate part W11c of the wire W11 is folded back at the distal end of the sheath main body 2, a substantial half W11b on the other end portion side of the wire W11 is inserted through the main lumen 22 formed in the sheath main body 2, and both end portions (one end portion and the other end portion) thereof are disposed on the proximal end side of the sheath main body 2. Also with this example illustrated in Figs. 6A to 6C, it is possible to reduce the number of wire lumens while achieving the same action and effect as the configuration illustrated in Figs. 2A to 2C.

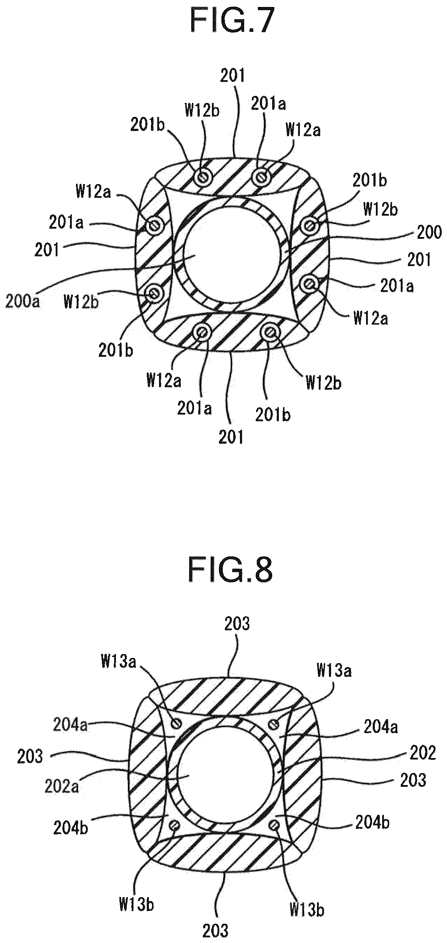

Further, although a plurality of wire lumens formed in the tube wall of the sheath main body 2 having the main lumen 22 have been exemplified in the embodiment described above, a single-lumen tube 200 having a main lumen 200a and at least one multi-lumen tube 201 having a plurality of wire lumens 201a and 201b may be combined and integrated with each other to be used as the sheath main body as illustrated in Fig. 7.

In the example illustrated in Fig. 7, four multi-lumen tubes 201 are disposed around the single-lumen tube 200, which has a substantially circular cross section and the main lumen 200a. The multi-lumen tube 201 has a substantially elliptical cross section and the pair of wire lumens 201a and 201b each having a substantially circular cross section. A substantial half W12a on one end portion side of a wire W12 (four in total) is inserted through the wire lumen 201a, and a substantial half W12b on the other end portion side of wire W12 is inserted through the wire lumen 201b. The single-lumen tube 200 and the multi-lumen tube 201 can be integrated with each other by, for example, adhesion by means of an adhesive or heat welding. Although it is not always easy to manufacture what has a plurality of wire lumens formed in the tube wall of the sheath main body 2 having the main lumen 22, it is possible to improve productivity by combining and integrating a plurality of tubes as described above.

Further, although the sheath main body is configured by the single-lumen tube 200 and the multi-lumen tube 201 being combined and integrated in the embodiment illustrated in Fig. 7, a single-lumen tube 202 having a main lumen 202a and a plurality of long members 203 may be combined and integrated, as illustrated in Fig. 8 and such that a gap is formed between the single-lumen tube 202 and the long member 203, to be used as the sheath main body. In this case, the gap may be used as wire lumens 204a and 204b.

In the example illustrated in Fig. 8, four solid long members 203 are disposed around the single-lumen tube 202, which has a substantially circular cross section and the main lumen 202a. The long member 203 has a substantially elliptical cross section. Four gaps are formed between the single-lumen tube 202 and two long members 203, each of the gaps has a substantially triangular cross section, and the four gaps are used as two pairs of wire lumens 204a and 204b. A substantial half W13a on one end portion side of a wire W13 (two in total) is inserted through the wire lumen 204a, and a substantial half W13b on the other end portion side of the wire W13 is inserted through the wire lumen 204b. The single-lumen tube 202 and the long member 203 can be integrated with each other by, for example, adhesion by means of an adhesive or heat welding. In this embodiment, no multi-lumen tube is necessary, and thus the productivity can be further improved.

The embodiments described above have been described so that the understanding of the invention is facilitated.

### EXPLANATIONS OF LETTERS OR NUMERALS

1 CATHETER SHEATH (MOVABLE CATHETER)
2 SHEATH MAIN BODY (TUBE)
21 DEFLECTION PORTION
22, 200a, 202aMAIN LUMEN
23a to 23d, 24a, 25a, 26a, 27a, 27b, 28a, 28b, 201a, 201b, 204a, 204b WIRE LUMEN
29 TIP PROTECTION MEMBER
200, 202 SINGLE-LUMEN TUBE
201 MULTI-LUMEN TUBE
203 LONG MEMBER
3 OPERATION PORTION
31 ROTATION OPERATION MEMBER
32 GRIP SECTION
4 GRIP PORTION
42 HOLDING PORTION
5 KNOB MEMBER
W1 TO W13 WIRE

## Claims

1. A movable catheter (1) provided with a tube (2) having a main lumen (22, 200a, 202a), wherein
the tube (2) has a plurality of wire lumens (23a - 23d, 24a, 25a, 26a, 201a, 201b, 204a, 204b) outside the main lumen (22, 200a, 202a), and the plurality of wire lumens (23a - 23d, 24a, 25a, 26a, 201a, 201b, 204a, 204b) are spaced apart from each other and reach a distal end portion of the tube (2) from a proximal end portion of the tube (2),
the movable catheter (1) includes at least two wires (W1 - W7, W12, W13),
in each of the at least two wires (W1 - W7, W12, W13), a substantial half (W1a - W7a, W12a, W13a) of the wire (W1 - W7, W12, W13) at one end portion side of the wire (W1 - W7, W12, W13) is inserted through one of the wire lumens (23a - 23d, 24a, 25a, 26a, 201a, 201b, 204a, 204b), an intermediate part (W1c- W4c) of the wire (W1 - W7, W12, W13) is folded back on the distal end portion of the tube (2), a substantial half (W1b - W7b, W12b, W13b) of the wire (W1 - W7, W12, W13) at the other end portion side of the wire is inserted through another one of the wire lumens (23a - 23d, 24a, 25a, 26a, 201a, 201b, 204a, 204b), and the one end portion and the other end portion of the wire (W1 - W7, W12, W13) are disposed at the proximal end side of the tube (2),
**characterized in that**
the movable catheter (1) further includes an operation portion (3) to which the one end portion and the other end portion of each of the at least two wires (W1 - W7, W12, W13) disposed at the proximal end side of the tube (2) are connected,
the operation portion (3) is configured so that it is possible to simultaneously pull both of the one end portion and the other end portion of each of the at least two wires (W1 - W7, W12, W13) and to simultaneously loosen both of the one end portion and the other end portion of each of the at least two wires (W1 - W7, W12, W13) by using the operation portion (3), and
when the operation portion simultaneously pull both of the one end portion and the other end portion of one wire of the at least two wires (W1 - W7, W12, W13), the operation portion simultaneously loosen both of the one end portion and the other end portion of the other wire of the at least two wires (W1 - W7, W12, W13), and when the operation portion simultaneously pull both of the one end portion and the other end portion of the other wire of the at least two wires (W1 - W7, W12, W13), the operation portion simultaneously loosen both of the one end portion and the other end portion of the one wire of the at least two wires (W1 - W7, W12, W13).

2. The movable catheter (1) according to claim 1, wherein the tube (2) comprises three or more wire lumens (23a, - 23d, 24a, 25a, 26a, 201a, 201b, 204a, 204b) and the movable catheter (1) comprises two or more wires (W1 - W7, W12, W13).

3. The movable catheter (1) according to claim 1 or 2, wherein the tube (2) has a single-lumen tube (200) having the main lumen (200a) and at least one multi-lumen tube (201) integrally disposed outside the single-lumen tube (200) and having a plurality of the wire lumens (201a, 201b).

4. A movable catheter (1) provided with a tube (2) having a main lumen (22), wherein
the tube (2) has a wire lumen (27a, 27b) outside the main lumen (22), and the wire lumen (27a, 27b) reaches a distal end portion of the tube (2) from a proximal end portion of the tube (2),
the movable catheter (1) includes a wire (W8, W9), a substantial half (W8a, W9a) of the wire (W8, W9) at one end portion side of the wire is inserted through the wire lumen (27a, 27b), an intermediate part (W8c, W9c) of the wire (W8, W9) is folded back on the distal end portion of the tube (2), a substantial half (W8b, W9b) of the wire (W8, W9) at the other end portion side of the wire is passed over an outside of the tube (2), and the one end portion and the other end portion of the wire (W8, W9) are disposed at the proximal end side of the tube (2), **characterized in that**
the movable catheter (1) further includes an operation portion (3) to which the one end portion and the other end portion of the wire (W8, W9) disposed at the proximal end side of the tube (2) are connected, it being possible to simultaneously pull both of the one end portion and the other end portion of the wire (W8, W9) by using the operation portion (3).

5. A movable catheter (1) provided with a tube (2) having a main lumen (22), wherein
the tube (2) has a wire lumen (28a, 28b) outside the main lumen (22), and the wire lumen (28a, 28b) reaches a distal end portion of the tube (2) from a proximal end portion of the tube (2),
the movable catheter (1) includes a wire (W10, W11), a substantial half (W10a, W11a) of the wire (W10, W11) at one end portion side of the wire is inserted through the wire lumen (28a, 28b), an intermediate part (W10c, W11c) of the wire (W10, W11) is folded back on the distal end portion of the tube (2), a substantial half (W10b, W11b) of the wire (W10, W11) of the other end portion side of the wire is inserted through the main lumen (22), and the one end portion and the other end portion of the wire (W10, W11) are disposed at the proximal end side of the tube (2),
**characterized in that**
the movable catheter (1) further includes an operation portion (3) to which the one end portion and the other end portion of the wire (W10, W11) disposed at the proximal end side of the tube (2) are connected, it being possible to simultaneously pull both of the one end portion and the other end portion of the wire (W10, W11) by using the operation portion (3).

## Patentansprüche

1. Beweglicher Katheter (1), der mit einem Schlauch (2) versehen ist, der ein Hauptlumen (22, 200a, 202a) aufweist, wobei
der Schlauch (2) eine Vielzahl von Drahtlumen (23a - 23d, 24a, 25a, 26a, 201a, 201b, 204a, 204b) außerhalb des Hauptlumens (22, 200a, 202a) aufweist und die mehreren Drahtlumen (23a - 23d, 24a, 25a, 26a, 201a, 201b, 204a, 204b) sind voneinander beabstandet und reichen von einem proximalen Endabschnitt des Schlauchs (2) bis zu einem distalen Endabschnitt des Schlauchs (2),
der bewegliche Katheter (1) umfasst mindestens zwei Drähte (W1 - W7, W12, W13),
in jedem der mindestens zwei Drähte (W1 - W7, W12, W13) erstreckt sich ein wesentlicher Teil (W1a - W7a, W12a, W13a) des Drahtes (W1 - W7, W12, W13) an einer Endabschnittsseite des Drahtes (W1 - W7, W12, W13) durch eines der Drahtlumen (23a - 23d, 24a, 25a, 26a, 201a, 201b, 204a, 204b) eingeführt ist, wobei ein mittlerer Abschnitt (W1c - W4c) des Drahtes (W1 - W7, W12, W13) wird am distalen Endabschnitt des Schlauchs (2) zurückgebogen, und ein wesentlicher Teil (W1b - W7b, W12b, W13b) des Drahts (W1 - W7, W12, W13) auf der Seite des anderen Endabschnitts des Drahtes wird durch ein weiteres der Drahtlumen (23a - 23d, 24a, 25a, 26a, 201a, 201b, 204a, 204b) eingeführt, und der eine Endabschnitt sowie der andere Endabschnitt des Drahtes (W1 - W7, W12, W13) befinden sich an der proximalen Endseite des Schlauchs (2),
**dadurch gekennzeichnet, dass**
der bewegliche Katheter (1) ferner einen Bedienungsabschnitt (3) umfasst, mit dem der eine Endabschnitt und der andere Endabschnitt jedes der mindestens zwei Drähte (W1 - W7, W12, W13), die an der proximalen Endseite des Schlauchs (2) angeordnet sind, verbunden sind,
der Bedienungsabschnitt (3) so ausgebildet ist, dass es möglich ist, sowohl den einen Endabschnitt als auch den anderen Endabschnitt jedes der mindestens zwei Drähte (W1 - W7, W12, W13) gleichzeitig zu ziehen und sowohl den einen Endabschnitt als auch den anderen Endabschnitt jedes der mindestens zwei Drähte (W1-W7, W12, W13) unter Verwendung des Bedienungsabschnitts (3) gleichzeitig zu lockern, und
wenn der Bedienungsabschnitt gleichzeitig sowohl den einen Endabschnitt als auch den anderen Endabschnitt eines Drahtes der mindestens zwei Drähte (W1 - W7, W12, W13) zieht, der Bedienungsabschnitt gleichzeitig sowohl den einen Endabschnitt als auch den anderen Endabschnitt des anderen Drahtes der mindestens zwei Drähte (W1 - W7, W12, W13) lockert, und wenn der Bedienungsabschnitt gleichzeitig sowohl an dem einen Endabschnitt als auch an dem anderen Endabschnitt des anderen Drahtes der mindestens zwei Drähte (W1 - W7, W12, W13) zieht, der Bedienungsabschnitt gleichzeitig sowohl den einen Endabschnitt als auch den anderen Endabschnitt des einen Drahtes der mindestens zwei Drähte (W1 - W7, W12, W13) lockert.

2. Beweglicher Katheter (1) nach Anspruch 1, wobei der Schlauch (2) drei oder mehr Drahtlumen (23a, - 23d, 24a, 25a, 26a, 201a, 201b, 204a, 204b) aufweist und der bewegliche Katheter (1) zwei oder mehr Drähte (W1 - W7, W12, W13) aufweist.

3. Beweglicher Katheter (1) nach Anspruch 1 oder 2, wobei der Schlauch (2) einen Einlumen-Schlauch (200) mit dem Hauptlumen (200a) und mindestens einen Mehrlumen-Schlauch (201) aufweist, das einstückig außerhalb des Einlumen-Schlauchs (200) angeordnet ist und eine Vielzahl von Drahtlumen (201a, 201b) aufweist.

4. Beweglicher Katheter (1), der mit einem Schlauch (2) versehen ist, der ein Hauptlumen (22) aufweist, wobei
der Schlauch (2) außerhalb des Hauptlumens (22) ein Drahtlumen (27a, 27b) aufweist und das Drahtlumen (27a, 27b) von einem proximalen Endabschnitt des Schlauchs (2) bis zu einem distalen Endabschnitt des Schlauchs (2) reicht,
der bewegliche Katheter (1) einen Draht (W8, W9) umfasst, wobei ein wesentlicher Teil (W8a, W9a) des Drahts (W8, W9) an einer Endabschnittsseite des Drahts durch das Drahtlumen (27a, 27b) eingeführt ist, ein mittlerer Abschnitt (W8c, W9c) des Drahts (W8, W9) am distalen Endabschnitt des Schlauchs (2) zurückgebogen ist, ein wesentlicher Teil (W8b, W9b) des Drahts (W8, W9) an der anderen Endabschnittsseite des Drahts über die Außenseite des Schlauchs (2) geführt ist, und der eine Endabschnitt sowie der andere Endabschnitt des Drahtes (W8, W9) an der proximalen Endseite des Schlauchs (2) angeordnet sind,
**dadurch gekennzeichnet, dass**
der bewegliche Katheter (1) ferner einen Bedienungsabschnitt (3) umfasst, mit dem der eine Endabschnitt und der andere Endabschnitt des Drahtes (W8, W9), die an der proximalen Endseite des Schlauchs (2) angeordnet sind, verbunden sind, wobei es möglich ist, sowohl den einen Endabschnitt als auch den anderen Endabschnitt des Drahtes (W8, W9) unter Verwendung des Bedienungsabschnitts (3) gleichzeitig zu ziehen.

5. Beweglicher Katheter (1), der mit einem Schlauch (2) mit einem Hauptlumen (22) versehen ist, wobei
der Schlauch (2) außerhalb des Hauptlumens (22) ein Drahtlumen (28a, 28b) aufweist und das Drahtlumen (28a, 28b) von einem proximalen Endabschnitt des Schlauchs (2) bis zu einem distalen Endabschnitt des Schlauchs (2) reicht,
der bewegliche Katheter (1) einen Draht (W10, W11) umfasst, wobei ein wesentlicher Teil (W10a, W11a) des Drahtes (W10, W11) an einem Endabschnitt des Drahtes durch das Drahtlumen (28a, 28b) eingeführt ist und ein mittlerer Abschnitt (W10c, W11c) des Drahtes (W10, W11) am distalen Endabschnitt des Schlauchs (2) zurückgebogen ist, ein wesentlicher Teil (W10b, W11b) des Drahtes (W10, W11) auf der Seite des anderen Endabschnitts durch das Hauptlumen (22) eingeführt ist, und der eine Endabschnitt sowie der andere Endabschnitt des Drahtes (W10, W11) an der proximalen Endseite des Schlauchs (2) angeordnet sind,
**dadurch gekennzeichnet, dass**
der bewegliche Katheter (1) ferner einen Bedienungsabschnitt (3) umfasst, mit dem der eine Endabschnitt und der andere Endabschnitt des Drahtes (W10, W11), die an der proximalen Endseite des Schlauchs (2) angeordnet sind, verbunden sind, wobei es möglich ist, sowohl den einen Endabschnitt als auch den anderen Endabschnitt des Drahtes (W10, W11) unter Verwendung des Bedienungsabschnitts (3) gleichzeitig zu ziehen.

## Revendications

1. Cathéter mobile (1) comprenant un tube (2) ayant une lumière principale (22, 200a, 202a), dans lequel
le tube (2) comprend une pluralité de lumières de fil (23a - 23d, 24a, 25a, 26a, 201a, 201b, 204a, 204b) à l'extérieur de la lumière principale (22, 200a, 202a), et la pluralité de lumières de fil (23a - 23d, 24a, 25a, 26a, 201a, 201b, 204a, 204b) sont espacées les unes des autres et atteignent une partie d'extrémité distale du tube (2) depuis une partie d'extrémité proximale du tube (2),
le cathéter mobile (1) comprend au moins deux fils (W1 - W7, W12, W13),
dans chacun des au moins deux fils (W1 - W7, W12, W13), une moitié substantielle (W1a - W7a, W12a, W13a) du fil (W1 - W7, W12, W13) du côté d'une extrémité du fil (W1 - W7, W12, W13) est insérée à travers l'une des lumières de fil (23a - 23d, 24a, 25a, 26a, 201a, 201b, 204a, 204b), une partie intermédiaire (W1c - W4c) du fil (W1 - W7, W12, W13) est repliée sur la partie d'extrémité distale du tube (2), une moitié substantielle (W1b - W7b, W12b, W13b) du fil (W1 - W7, W12, W13) du côté de l'autre extrémité du fil est insérée à travers une autre des lumières de fil (23a - 23d, 24a, 25a, 26a, 201a, 201b, 204a, 204b), et l'une des extrémités et l'autre extrémité du fil (W1 - W7, W12, W13) sont disposées du côté proximal du tube (2),
**caractérisé en ce que**
le cathéter mobile (1) comprend en outre une partie d'opération (3) à laquelle l'une des extrémités et l'autre extrémité de chacun des au moins deux fils (W1 - W7, W12, W13) disposés du côté proximal du tube (2) sont connectés,
la partie d'opération (3) est configurée de sorte qu'il est possible de tirer simultanément à la fois l'une des extrémités et l'autre extrémité de chacun des au moins deux fils (W1 - W7, W12, W13) et de relâcher simultanément à la fois l'une des extrémités et l'autre extrémité de chacun des au moins deux fils (W1 - W7, W12, W13) en utilisant la partie d'opération (3), et
lorsque la partie d'opération tire simultanément à la fois l'une des extrémités et l'autre extrémité d'un fil des au moins deux fils (W1 - W7, W12, W13), la partie d'opération relâche simultanément à la fois l'une des extrémités et l'autre extrémité de l'autre fil des au moins deux fils (W1 - W7, W12, W13), et lorsque la partie d'opération tire simultanément à la fois l'une des extrémités et l'autre extrémité de l'autre fil des au moins deux fils (W1 - W7, W12, W13), la partie d'opération relâche simultanément à la fois l'une des extrémités et l'autre extrémité de l'un des fils des au moins deux fils (W1 - W7, W12, W13).

2. Cathéter mobile (1) selon la revendication 1, dans lequel le tube (2) comprend trois ou plus de trois lumières de fil (23a, - 23d, 24a, 25a, 26a, 201a, 201b, 204a, 204b) et le cathéter mobile (1) comprend deux ou plus de deux fils (W1 - W7, W12, W13).

3. Cathéter mobile (1) selon l'une quelconque des revendications 1 ou 2, dans lequel le tube (2) comprend un tube à lumière unique (200) ayant la lumière principale (200a) et au moins un tube à lumières multiples (201) disposé de manière intégrée à l'extérieur du tube à lumière unique (200) et ayant une pluralité des lumières de fil (201a, 201b).

4. Cathéter mobile (1) comprenant un tube (2) ayant une lumière principale (22), dans lequel
le tube (2) comprend une lumière de fil (27a, 27b) à l'extérieur de la lumière principale (22), et la lumière de fil (27a, 27b) atteint une partie d'extrémité distale du tube (2) depuis une partie d'extrémité proximale du tube (2),
le cathéter mobile (1) comprend un fil (W8, W9), une moitié substantielle (W8a, W9a) du fil (W8, W9) du côté d'une extrémité du fil est insérée à travers la lumière de fil (27a, 27b), une partie intermédiaire (W8c, W9c) du fil (W8, W9) est repliée sur la partie d'extrémité distale du tube (2), une moitié substantielle (W8b, W9b) du fil (W8, W9) du côté de l'autre extrémité du fil est passée sur l'extérieur du tube (2), et l'une des extrémités et l'autre extrémité du fil (W8, W9) sont disposées du côté proximal du tube (2),
**caractérisé en ce que**
le cathéter mobile (1) comprend en outre une partie d'opération (3) à laquelle l'une des extrémités et l'autre extrémité du fil (W8, W9) disposées du côté proximal du tube (2) sont connectées, étant possible de tirer simultanément à la fois l'une des extrémités et l'autre extrémité du fil (W8, W9) en utilisant la partie d'opération (3).

5. Cathéter mobile (1) comprenant un tube (2) ayant une lumière principale (22), dans lequel
le tube (2) comprend une lumière de fil (28a, 28b) à l'extérieur de la lumière principale (22), et la lumière de fil (28a, 28b) atteint une partie d'extrémité distale du tube (2) depuis une partie d'extrémité proximale du tube (2),
le cathéter mobile (1) comprend un fil (W10, W11), une moitié substantielle (W10a, W11a) du fil (W10, W11) du côté d'une extrémité du fil est insérée à travers la lumière de fil (28a, 28b), une partie intermédiaire (W10c, W11c) du fil (W10, W11) est repliée sur la partie d'extrémité distale du tube (2), une moitié substantielle (W10b, W11b) du fil (W10, W11) du côté de l'autre extrémité du fil est insérée à travers la lumière principale (22), et l'une des extrémités et l'autre extrémité du fil (W10, W11) sont disposées du côté proximal du tube (2),
**caractérisé en ce que**
le cathéter mobile (1) comprend en outre une partie d'opération (3) à laquelle l'une des extrémités et l'autre extrémité du fil (W10, W11) disposées du côté proximal du tube (2) sont connectées, étant possible de tirer simultanément à la fois l'une des extrémités et l'autre extrémité du fil (W10, W11) en utilisant la partie d'opération (3).
